# EUROPÄISCHE PATENTSCHRIFT

(11) **EP 1 392 670 B1**
(45) Veröffentlichungstag und Bekanntmachung des Hinweises auf die Patenterteilung: **20.07.2005**
(21) Anmeldenummer: 02743057.8
(22) Anmeldetag: 21.05.2002
(51) Int. Cl.: C07D 307/68, A61K 31/341, A61P 29/02

(54) **SUBSTITUIERTE C-FURAN-2-YL-METHYLAMIN- UND C-THIOPHEN-2-YL-METHYLAMIN-DERIVATE**
SUBSTITUTED C-FURAN-2-YL-METHYLAMINE AND C-THIOPHEN-2-YL-METHYLAMINE DERIVATIVES
DERIVES SUBSTITUES DE C-FURAN-2-YL-METHYLAMINE ET C-THIOPHEN-2-YL-METHYLAMINE

(30) Priorität: 22.05.2001 DE 10125145
(43) Veröffentlichungstag der Anmeldung: 03.03.2004
(73) Patentinhaber: Grünenthal GmbH, 52078 Aachen (DE)
(72) Erfinder: MAUL, Corinna, 52066 Aachen (DE); PRZEWOSNY, Michael, 52062 Aachen (DE)
(86) Internationale Anmeldenummer: PCT/EP2002/005542
(87) Internationale Veröffentlichungsnummer: WO 2002/094802

(56) Entgegenhaltungen:
- WO-A-01/68616
- WO-A-02/44171
- US-A- 3 816 457
- US-A- 3 890 445
- PETASIS N A ET AL: "One-step three-component reaction among organoboronic acids, amines and salicylaldehydes" TETRAHEDRON LETTERS, ELSEVIER SCIENCE PUBLISHERS, AMSTERDAM, NL, Bd. 42, Nr. 4, 22. Januar 2001 (2001-01-22), Seiten 539-542, XP004314729 ISSN: 0040-4039

## Beschreibung

Die vorliegende Erfindung betrifft substituierte C-Furan-2-yl-methylamin- und C-Thiophen-2-yl-methylamin-Derivate, Verfahren zu ihrer Herstellung, sie enthaltende Arzneimittel und pharmazeutische Zusammensetzungen sowie ihre Verwendung zur Herstellung von Schmerzmitteln, zur Herstellung eines Lokalanästhetikums, eines Antiarrhythmikums, eines Antiemetikums, eines Nootropikums und/oder eines Arzneimittels zur Behandlung und/oder Prophylaxe von cardiovaskulären Erkrankungen, von Harninkontinenz, Diarrhöe, Pruritus und/oder Entzündungen und/oder eines Arzneimittels zur Behandlung von Depressionen und/oder Alkohol- und/oder Drogen- und/oder Medikamentenmißbrauch und/oder eines Arzneimittels zur Vigilanzsteigerung.

Die Behandlung chronischer und nicht chronischer Schmerzzustände hat in der Medizin eine große Bedeutung. Es besteht ein weltweiter Bedarf an gut wirksamen Therapien für eine patientengerechte und zielorientierte Behandlung chronischer und nicht chronischer Schmerzzustände, wobei hierunter die erfolgreiche und zufriedenstellende Schmerzbehandlung für den Patienten zu verstehen ist.

Klassische Opioide wie Morphin sind bei der Therapie starker bis stärkster Schmerzen gut wirksam. Ihr Einsatz wird jedoch durch die bekannten Nebenwirkungen, wie z.B. Atemdepression, Erbrechen, Sedierung, Obstipation und Toleranzentwicklung, limitiert. Außerdem sind sie bei neuropathischen oder inzidentiellen Schmerzen, unter denen insbesondere Tumorpatienten leiden, weniger wirksam.

Opioide entfalten ihre analgetische Wirkung durch Bindung an zellmebranständige Rezeptoren, die zu der Familie der so genannten G-Proteingekoppelten Rezeptoren gehören. Neben diesen gibt es weitere Rezeptoren sowie Ionenkanäle, die wesentlich an dem System der Schmerzentstehung und der Schmerzweiterleitung beteiligt sind, beispielsweise die sogenannte Batrachotoxin-(BTX-)Bindungsstelle (= Bindungsstelle 2) des Natriumkanals.

Der vorliegenden Erfindung liegt als eine Aufgabe zugrunde, analgetisch wirksame Verbindungen zur Verfügung zu stellen, die sich zur Schmerztherapie - ggf. auch zur Therapie chronischer und neuropathischer Schmerzen - eignen. Darüber hinaus sollten diese Substanzen möglichst keine der Nebenwirkungen, die üblicherweise bei der Anwendung von Opioiden wie Morphin auftreten, wie z.B. Übelkeit, Erbrechen, Abhängigkeit, Atemdepression oder Obstipation, hervorrufen.

Die Aufgabe wird durch Verbindungen der allgemeinen Formel (I) gelöst, die analgetisch wirksam sind und eine hohe Affinität zu der BTX-Bindungsstelle des Natriumkanals aufweisen. Bei den erfindungsgemäßen Verbindungen handelt es sich um substituierte C-Furan-2-yl-methylamin und C-Thiophen-2-yl-methylamin-Derivate der Formel (I) 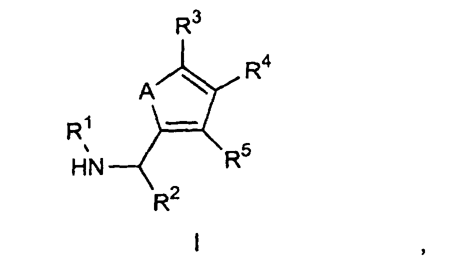 worin
- A: O oder S bedeutet;
- R¹: für 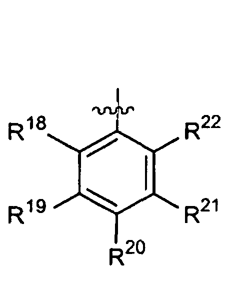 oder für 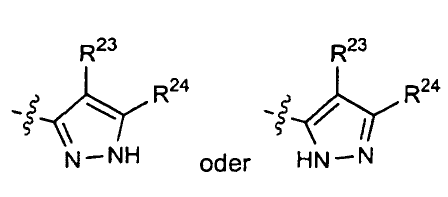 steht;
- R²: -(C=O)-Phenyl oder -*cyclo*-C₃H₄R¹⁷ bedeutet;
- R³, R⁴ und R⁵: unabhängig voneinander H, Methyl, -CH₂-OH, -CH₂-S-CH₃ oder -CH₂-S-CH₂-furan-2-yl, -C(=O)OMethyl, -C(=O)OEthyl, -CH₂-C(=O)OEthyl bedeuten;
- R¹⁷: -C(=O)OH oder -C(=O)O-C₁₋₆-Alkyl bedeutet; und
- R¹⁸, R¹⁹, R²⁰, R²¹, R²², R²³ und R²⁴: unabhängig voneinander H, OH, SH, -O-C₁₋₆-Alkyl, -OAryl, -S-C₁₋₆-Alkyl, -SAryl, F, Cl, Br, I, -CN, C₁₋₆-Alkyl, CF₃, CO(=O)H, CO(=O)-C₁₋₆-Alkyl oder -N=N-Aryl bedeuten,
in Form ihrer Racemate, in Form der reinen Enantiomeren oder Diastereomeren oder in Form von Mischungen der Enantiomeren oder Diastereomeren in einem beliebigen Mischungsverhältnis.

Folgende Racemate von Verbindungen sind als solche im Stand der Technik beschrieben, ohne daß ihre Verwendung in einem Arzneimittel oder zur Herstellung irgendeines Arzneimittels offenbart werden würde:
1,2-Di-2-furanyl-2-(phenylamino)-ethanon (K. G. Lewis und C. E. Mulquiney, *Aust. J. Chem*. (1990), 655-663);
1,2-Di-2-furanyl-2-[(4-methylphenyl)amino]-ethanon (K. Heyms und W. Stumme, *Chem. Ber.* (1956) 2833-2844);
1,2-Di-2-furanyl-2-(pyrazinylamino)-ethanon;
5-Chlor-N-[cyclopropyl[5-(2-ethoxyethyl)-2-thienyl]methyl]-6-ethyl-4-pyridinamin und

Ferner sind im Stand der Technik folgende Racemate von Verbindungen der allgemeinen Formel (I) und ihre Verwendung als Arzneimittel oder zur Herstellung von Arzneimitteln beschrieben:
N-(Cyclopropyl-2-thienylmethyl)-3,4,5,6-tetrahydro-2-pyridinamin,
N-(Cyclopropyl-2-thienylmethyl)-3,4,5,6-tetrahydro-2H-azepinamin und
N-(Cyclopropyl-2-thienylmethyl)-3,4,5,6-tetrahydro-2-azocinamin (US 3,890,445; US 3,816,457; J. M. Grisar et al., *J..Med. Chem.* (1976) 365-369).

Die Begriffe "Alkyl", "C₁₋₁₂-Alkyl", "C₁₋₈-Alkyl" bzw. "C₁₋₆-Alkyl" umfassen im Sinne dieser Erfindung acyclische gesättigte oder ungesättigte Kohlenwasserstoffreste, die verzweigt oder geradkettig sowie unsubstituiert oder einfach substituiert oder mehrfach gleich oder verschieden substituiert sein können, mit (wie im Fall von C₁₋₁₂-Alkyl) 1 bis 12 (d.h. 1, 2, 3, 4, 5, 6, 7, 8, 9, 10, 11 oder 12), mit (wie im Fall von C₁₋₁₂-Alkyl) 1 bis 8 (d.h. 1, 2, 3, 4, 5, 6, 7 oder 8) bzw. mit (wie im Fall von C₁₋₆-Alkyl) 1 bis 6 (d.h. 1, 2, 3, 4, 5 oder 6) C-Atomen, d.h. C₁₋₁₂-Alkanyle, C₁₋₈-Alkanyle bzw. C₁₋₆-Alkanyle, C₂₋₁₂-Alkenyle, C₂₋₈-Alkenyle bzw. C₂₋₆-Alkenyle und C₂₋₁₂-Alkinyle, C₂₋₈-Alkinyle bzw. C₂₋₆-Alkinyle. Dabei weisen "Alkenyle" mindestens eine C-C-Doppelbindung und "Alkinyle" mindestens eine C-C-Dreifachbindung auf, während "Alkanyle" gesättigt sind. Vorteilhaft steht Alkyl für Alkanyl und ist aus der Gruppe ausgewählt, die Methyl, Ethyl, n-Propyl, 2-Propyl, n-Butyl und tert-Butyl umfaßt.

"C₃₋₈-Cycloalkyl" (bzw. "Cycloalkyl") bedeutet im Sinne dieser Erfindung einen cyclischen gesättigten oder ungesättigten Kohlenwasserstoff-Rest mit 3, 4, 5, 6, 7 oder 8 C-Atomen, wobei der Rest unsubstituiert oder einfach substituiert oder mehrfach gleich oder verschieden substituiert und ggf. benzokondensiert sein kann. Beispielhaft steht Cycloalkyl für Cyclopropyl, Cyclobutyl, Cyclopentyl, Cyclohexyl, Cycloheptyl, Cyclooctyl, Cyclopentenyl, Cyclohexenyl, Cycloheptenyl und Cyclooctenyl. Für die Zwecke der vorliegenden Erfindung besonders bevorzugt sind Cyclopropyl, Cyclopropyl-2-carbonsäure und Cyclopropyl-2-carbonsäureethylester. Der ggf. substituierte Cyclopropylrest wird auch als *cyclo*-C₃H₅ bezeichnet.

Unter dem Ausdruck "Aryl" ist für die Zwecke der vorliegenden Erfindung ein Rest zu verstehen, der aus der Gruppe, die Phenyl, Naphthyl, Anthracenyl und Biphenyl umfaßt, ausgewählt ist und unsubstituiert oder einfach oder mehrfach gleich oder verschieden substituiert ist. Die ArylReste können auch mit weiteren gesättigten, (partiell) ungesättigten oder aromatischen Ringsystemen kondensiert sein. Jeder Aryl-Rest kann unsubstituiert oder einfach oder mehrfach substituiert vorliegen, wobei die Aryl-Substituenten gleich oder verschieden und in jeder beliebigen Position des Aryls sein können. Vorteilhafterweise steht Aryl für einen unsubstituierten oder ein- oder mehrfach gleich oder verschieden substituierten Phenylrest.

Der Ausdruck "Heterocyclyl" steht für einen monocyclischen oder polycyclischen organischen Rest, in dem mindestens ein Cyclus 1 Heteroatom oder 2, 3, 4 oder 5 gleiche oder verschiedene Heteroatome enthält, das/die aus der Gruppe, die N, O und S enthält, ausgewählt ist/sind, wobei der Rest gesättigt oder ungesättigt ist und unsubstituiert oder einfach substituiert oder mehrfach gleich oder verschieden substituiert ist. Beispiele für Heterocyclyl-Reste im Sinne dieser Erfindung sind monocyclische fünf-, sechs- oder siebengliedrige organische Reste mit 1 Heteroatom oder 2, 3, 4 oder 5 gleichen oder verschiedenen Heteroatomen, bei dem/denen es sich um Stickstoff, Sauerstoff und/oder Schwefel handelt, und deren benzokondensierte Analoga. Eine Untergruppe der Heterocyclyl-Reste bilden die "Heteroaryl"-Reste, bei denen es sich um solche Heterocyclyle handelt, in denen der mindestens eine Cyclus, der das/die Heteroatom/e enthält, heteroaromatisch ist. Jeder Heteroaryl-Rest kann unsubstituiert oder einfach substituiert oder mehrfach gleich oder verschieden substituiert vorliegen. Beispiele für Heterocyclyl-Reste im Sinne der vorliegenden Erfindung sind Pyrrolidinyl, Tetrahydrofuryl, Piperidinyl, Piperazinyl und insbesondere Morpholinyl. Beispiele für Heterocyclyle, die zugleich Heteroaryl-Reste darstellen, sind Pyrrolyl, Imidazolyl, Pyridazinyl, Pyrimidinyl, Pyrazinyl, Furanyl, Thienyl, Pyridinyl und insbesondere Pyrazolyl sowie deren benzokondensierte Analoga. Alle diese Reste können jeweils unsubstituiert oder einfach oder mehrfach gleich oder verschieden substituiert vorliegen. Wenn -NR¹⁵R¹⁶ zusammen einen Heterocyclyl-Ring bilden, so ist dieser Ring vorzugsweise ein Pyrrolidinyl-, Piperidinyl-, Piperazinyl- oder Morpholinyl-Rest.

Die Ausdrücke "(C₁₋₆-Alkyl)-C₃₋₈-Cycloalkyl", "(C₁₋₆-Alkyl)-Heterocyclyl" und "(C₁₋₆-Alkyl)-Aryl" bedeuten für die Zwecke der vorliegenden Erfindung, daß der Cycloalkyl-, Heterocyclyl- bzw. Aryl-Rest über eine C₁₋₆-Alkyl-Gruppe an die mit ihm substituierte Verbindung gebunden ist.

Im Zusammenhang mit "Alkyl", "Alkanyl", "Alkenyl", "Alkinyl" und "Cycloalkyl" versteht man unter dem Begriff "substituiert" im Sinne dieser Erfindung die Substitution eines Wasserstoffatoms durch beispielsweise F, Cl, Br, I, -CN, -NC, NH₂, NH-Alkyl, NH-Aryl, NH-Alkyl-Aryl, NH-Heterocyclyl, NH-Alkyl-OH, N(Alkyl)₂, N(Alkyl-Aryl)₂, N(Heterocyclyl)₂, N(Alkyl-OH)₂, NO, NO₂, SH, S-Alkyl, S-Aryl, S-Alkyl-Aryl, S-Heterocyclyl, S-Alkyl-OH, S-Alkyl-SH, OH, O-Alkyl, O-Aryl, O-Alkyl-Aryl, O-Heterocyclyl, O-Alkyl-OH, CHO, C(=O)C₁₋₆-Alkyl, C(=S)C₁₋₆-Alkyl, C(=O)Aryl, C(=S)Aryl, C(=O)C₁₋₆-Alkyl-Aryl, C(=S)C₁₋₆-Alkyl-Aryl, C(=O)-Heterocyclyl, C(=S)-Heterocyclyl, CO₂H, CO₂-Alkyl, CO₂-Alkyl-Aryl, C(=O)NH₂, C(=O)NH-Alkyl, C(=O)NHAryl, C(=O)NH-Heterocyclyl, C(=O)N(Alkyl)₂, C(=O)N(Alkyl-Aryl)₂, C(=O)N(Heterocyclyl)₂, SO-Alkyl, SO₂-Alkyl, SO₂-Alkyl-Aryl, SO₂NH₂, SO₃H, SO₃-Alkyl, Cycloalkyl, Aryl oder Heterocyclyl, wobei unter mehrfach substituierten Resten solche Reste zu verstehen sind, die entweder an verschiedenen oder an gleichen Atomen mehrfach, z.B. zwei- oder dreifach, substituiert sind, beispielsweise dreifach am gleichen C-Atom wie im Falle von CF₃ oder -CH₂CF₃ oder an verschiedenen Stellen wie im Falle von -CH(OH)-CH=CCl-CH₂Cl. Die Mehrfachsubstitution kann mit dem gleichen oder mit verschiedenen Substituenten erfolgen. Besonders bevorzugt für die Zwecke der vorliegenden Erfindung sind CF₃, -CH₂-OH und -CH₂-C(=O)OEthyl als substituiertes Alkyl sowie Cyclopropyl-2-carbonsäure und Cyclopropyl-2-carbonsäureethylester als substituiertes Cycloalkyl.

In Bezug auf "Aryl", "Heterocyclyl" sowie "Heteroaryl" versteht man im Sinne dieser Erfindung unter "einfach substituiert" oder "mehrfach substituiert" die ein- oder mehrfache, z.B. zwei-, drei- oder vierfache, Substitution eines oder mehrerer Wasserstoffatome des Ringsystems durch einen geeigneten Substituenten. Soweit die Bedeutung dieser geeigneten Substituenten im Zusammenhang mit "Aryl", "Heterocyclyl" oder "Heteroaryl" nicht an anderer Stelle der Beschreibung oder in den Ansprüchen definiert ist, sind geeignete Substituenten F, Cl, Br, I, -CN, -NC, NH₂, NH-Alkyl, NH-Aryl, NH-Alkyl-Aryl, NH-Heterocyclyl, NH-Alkyl-OH, N(Alkyl)₂, N(Alkyl-Aryl)₂, N(Heterocyclyl)₂, N(Alkyl-OH)₂, NO, NO₂, SH, S-Alkyl, S-Cycloalkyl, S-Aryl, S-Alkyl-Aryl, S-Heterocyclyl, S-Alkyl-OH, S-Alkyl-SH, OH, O-Alkyl, O-Cycloalkyl, O-Aryl, O-Alkyl-Aryl, O-Heterocyclyl, O-Alkyl-OH, CHO, C(=O)C₁₋₆-Alkyl, C(=S)C₁₋₆-Alkyl, C(=O)Aryl, C(=S)Aryl, C(=O)-C₁₋₆-Alkyl-Aryl, C(=S)C₁₋₆-Alkyl-Aryl, C(=O)-Heterocyclyl, C(=S)-Heterocyclyl, CO₂H, CO₂-Alkyl, CO₂-Alkyl-Aryl, C(=O)NH₂, C(=O)NH-Alkyl, C(=O)NHAryl, C(=O)NH-Heterocyclyl, C(=O)N(Alkyl)₂, C(=O)N(Alkyl-Aryl)₂, C(=O)N(Heterocyclyl)₂, S(O)-Alkyl, S(O)-Aryl, SO₂-Alkyl, SO₂-Aryl, SO₂NH₂, SO₃H, CF₃, =O, =S; Alkyl, Cycloalkyl, Aryl und/oder Heterocyclyl; an einem oder ggf. verschiedenen Atomen (wobei ein Substituent ggf. seinerseits substituiert sein kann). Die Mehrfachsubstitution erfolgt dabei mit dem gleichen oder mit unterschiedlichen Substituenten.

"Benzokondensiert" bedeutet für die Zwecke der vorliegenden Erfindung, daß ein Benzol-Ring an einen anderen Cyclus ankondensiert ist.

Pharmazeutisch annehmbare Salze im Sinne dieser Erfindung sind solche Salze der erfindungsgemäßen Verbindungen gemäß der allgemeinen Formel (I), die bei pharmazeutischer Verwendung physiologisch - insbesondere bei Anwendung am Säugetier und/oder Menschen - verträglich sind. Solche pharmazeutisch annehmbaren Salze können beispielsweise mit anorganischen oder organischen Säuren oder für den Fall, daß die erfindungsgemäßen Verbindungen Carbonsäuren sind, mit Basen gebildet werden.

Vorzugsweise werden die pharmazeutisch annehmbaren Salze der erfindungsgemäßen Verbindungen gemäß der allgemeinen Formel (I) mit Salzsäure, Bromwasserstoffsäure, Schwefelsäure, Phosphorsäure, Methansulfonsäure, p-Toluolsulfonsäure, Kohlensäure, Ameisensäure, Essigsäure, Oxalsäure, Bernsteinsäure, Weinsäure, Mandelsäure, Fumarsäure, Milchsäure, Citronensäure, Glutaminsäure oder Asparaginsäure gebildet. Handelt es sich bei den erfindungsgemäßen Verbindungen um Carbonsäuren, können die pharmazeutisch annehmbaren Salze auch durch Umsetzung mit Basen, wie z.B. Natriumhydrogencarbonat oder Natriumcarbonat, gebildet werden. Bei den gebildeten Salzen handelt es sich u.a. um Hydrochloride, Hydrobromide, Phosphate, Carbonate, Hydrogencarbonate, Formiate, Acetate, Oxalate, Succinate, Tartrate, Fumarate, Citrate und Glutaminate bzw. um NatriumSalze. Ebenfalls bevorzugt sind die Hydrate der erfindungsgemäßen Verbindungen, die z.B. durch Kristallisation aus wäßriger Lösung erhalten werden können.

Alle erfindungsgemäßen Verbindungen enthalten mindestens ein Asymmetriezentrum, nämlich das mit R² und NH-R¹ substituierte Kohlenstoffatom der Formel (I). Daher können die erfindungsgemäßen Verbindungen der allgemeinen Formel (I) in Form ihrer Racemate, in Form der reinen Enantiomeren und/oder Diastereomeren oder in Form von Mischungen dieser Enantiomeren bzw. Diastereomeren vorliegen, und zwar sowohl in Substanz als auch als pharmazeutisch annehmbare Salze dieser Verbindungen. Die Mischungen können in jedem beliebigen Mischungsverhältnis der Stereoisomeren vorliegen. Bevorzugt liegen die Verbindungen der allgemeinen Struktur (I) als enantiomerenreine Verbindungen vor.

Ganz besonders bevorzugt sind unter diesen solche Verbindungen der allgemeinen Formel (I), worin
- R²: -(C=O)-Phenyl oder -*cyclo*-C₃H₄-C(=O)OEthyl bedeutet;
- R³: H, Methyl, -CH₂-S-CH₃, -CH₂-S-CH₂-furan-2-yl oder -CH₂-C(=O)OEthyl bedeutet;
- R⁴: H, Methyl, -CH₂-OH, -C(=O)OMethyl oder -C(=O)OEthyl bedeutet;
- R⁵: H bedeutet;
- R¹⁸, R¹⁹, R²⁰, R²¹ und R²²: unabhängig voneinander H, -OPhenyl, F, Cl, Br, -CN, Methyl und CF₃ bedeuten, wobei mindestens 3 der Reste R¹⁸, R¹⁹, R²⁰, R²¹ und R²² H bedeuten; und
- R²³ und R²⁴: unabhängig voneinander H, OH, -S-Methyl, -CN, CO(=O)-Ethyl oder -N=N-Phenyl bedeuten.

Beispielhafte und vorteilhafte Verbindungen der vorliegenden Erfindung sind aus der Gruppe ausgewählt, die
5-[1-(2-Chlor-phenylamino)-2-oxo-2-phenyl-ethyl]-2-methyl-furan-3-carbonsäureethylester;
5-[1-(4-Chlor-2-methyl-phenylamino)-2-oxo-2-phenyl-ethyl]-2-methylfuran-3-carbonsäuremethylester;
5-[1-(4-Chlor-2-fluor-phenylamino)-2-oxo-2-phenyl-ethyl]-2-methylfuran-3-carbonsäuremethylester; und
5-[1-(4-Chlor-2-methyl-phenylamino)-2-oxo-2-phenyl-ethyl]-2-methylfuran-3-carbonsäureethylester;
umfaßt sowie ihre pharmazeutisch annehmbaren Salze, insbesondere ihre Hydrochloride.

Die vorliegende Erfindung betrifft ferner ein Verfahren zur Herstellung einer Verbindung der allgemeinen und wie oben definerten Formel (I), das dadurch gekennzeichnet ist, daß ein Amin der allgemeinen Formel (II) 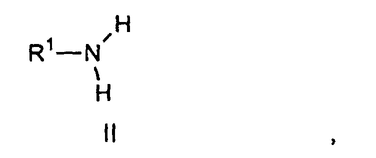 worin R¹ wie oben definiert ist,
mit einem Aldehyd der allgemeinen Formel (III) 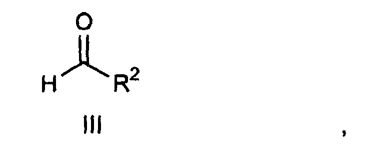 worin R² wie oben definiert ist,
und einem Heterocyclus der allgemeinen Formel (IV) 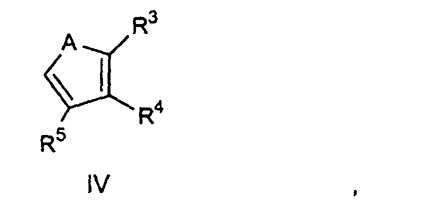 worin A, R³, R⁴ und R⁵ wie oben definiert sind,
in Gegenwart einer Säure umgesetzt werden.

Bei der eingesetzten Säure handelt es sich um eine anorganische oder organische Protonen- oder Lewis-Säure. Bevorzugt wird die Reaktion in Gegenwart einer organischen Säure, z.B. Essigsäure, Trifluoressigsäure oder Methansulfonsäure, insbesondere Trifluoressigsäure, durchgeführt.

Das erfindungsgemäße Herstellungsverfahren kann in jedem geeigneten Lösungsmittel durchgeführt werden, in dem die Reaktanten sich ausreichend lösen. Bevorzugt sind als Lösungsmittel organische Solventien, z.B. Dichlormethan oder insbesondere Acetonitril.

Die erfindungsgemäßen Verfahren werden zweckmäßig bei einer Temperatur von 0 bis 100 °C, insbesondere bei 15 bis 50°C durchgeführt. Die Reaktionszeit beträgt vorzugsweise 15 Minuten bis 12 Stunden und kann den jeweiligen Erfordernissen angepaßt werden.

Alle in den erfindungsgemäßen Verfahren eingesetzten Amine der allgemeinen Struktur (II), die Aldehyde der allgemeinen Struktur (III) und die Thiophene bzw. Furane der allgemeinen Struktur (IV) sind käuflich erhältlich oder können nach im Stand der Technik allgemein bekannten Verfahren hergestellt werden.

Das erfindungsgemäße Verfahren kann auch in semi- oder vollautomatisierter Form als Parallelsynthese einer Gruppe von erfindungsgemäßen Verbindungen der allgemeinen Formel (I) durchgeführt werden, so daß auch Substanzbibliotheken ohne weiteres hergestellt werden können, die mindestens eine Verbindung und vorzugsweise mindestens 80 und insbesondere 160 erfindungsgemäße Verbindungen der allgemeinen Formel (I) (oder ein ganzzahliges Vielfaches davon) enthalten.

Für die Zwecke der vorliegenden Erfindung wird unter einer "Substanzbibliothek" eine Gruppe von Verbindungen verstanden, die nach dem gleichen Verfahren unter gleichen oder nahezu gleichen Reaktionsbedingungen und unter Variation eines Reagenzes oder mehrerer Reagenzien hergestellt werden. Eine solche Substanzbibliothek kann die Bibliotheksmitglieder sowohl als einzelne reine Verbindungen als auch als Mischung dieser Verbindungen enthalten. Mit Hilfe dieser Substanzbibliothek kann beispielsweise ein medizinisches Screening in einem oder mehreren in-vitro-Screening-Verfahren in automatisierter Form durchgeführt werden.

Die erfindungsgemäßen Verbindungen der allgemeinen Formel (I) können sowohl in Substanz als auch als Salz isoliert werden. Die Substanzen der allgemeinen Formel (I) werden üblicherweise nach Umsetzung gemäß dem oben dargelegten erfindungsgemäßen Verfahren und anschließender herkömmlicher Aufarbeitung erhalten. Die so gewonnenen Verbindungen können dann beispielsweise durch Versetzen mit einer anorganischen oder organischen Säure, vorzugsweise mit Salzsäure, Bromwasserstoffsäure, Schwefelsäure, Phosphorsäure, Methansulfonsäure, p-Toluolsulfonsäure, Kohlensäure, Ameisensäure, Essigsäure, Oxalsäure, Bernsteinsäure, Weinsäure, Mandelsäure, Fumarsäure, Milchsäure, Citronensäure, Glutaminsäure oder Asparaginsäure, in das korrespondierende Salz überführt werden. Bei den gebildeten Salzen handelt es sich u.a. um Hydrochloride, Hydrobromide, Phosphate, Carbonate, Hydrogencarbonate, Formiate, Acetate, Oxalate, Succinate, Tartrate, Fumarate, Citrate und Glutaminate. Soweit es sich bei den erfindungsgemäßen Verbindungen der allgemeinen Formel (I) um Carbonsäuren handelt, kann die Salzbildung durch Zugabe einer physiologisch verträglichen Base, z.B. NaHCO₃ oder Natriumcarbonat, herbeigeführt werden; für die Carbonsäuren ist insbesondere die Bildung des Natriumsalzes bevorzugt. Die besonders bevorzugte Hydrochloridbildung kann insbesondere auch durch Versetzen der in einem geeigneten organischen Lösungsmittel gelösten Base (I) mit Trimethylsilylchlorid (TMSCI) herbeigeführt werden.

Soweit die Verbindungen der allgemeinen Formel (I) in dem erfindungsgemäßen Herstellungsverfahren als Racemate oder als Mischungen ihrer verschiedenen Enantiomeren und/oder Diastereomeren erhalten werden, können diese Mischungen nach im Stand der Technik wohlbekannten Verfahren aufgetrennt werden. Geeignete Methoden sind u.a. chromatographische Trennverfahren, insbesondere Flüssigkeitschromatographie-Verfahren unter Normal- oder erhöhtem Druck, bevorzugt MPLC- und HPLC-Verfahren, sowie Verfahren der fraktionierten Kristallisation. Dabei können insbesondere einzelne Enantiomeren z.B. mittels HPLC an chiraler Phase oder mittels Kristallisation von mit chiralen Säuren, etwa (+)-Weinsäure, (-)-Weinsäure oder (+)-10-Camphersulfonsäure, oder - sofern es sich um Säuren handelt - mit chiralen Basen, etwa Brucin oder (-)-Ephedrin, gebildeten diastereomeren Salzen voneinander getrennt werden.

Darüber hinaus ist ein Arzneimittel, das mindestens eine der erfindungsgemäßen und wie oben definierten Verbindungen der allgemeinen Formel (I) bzw. eines ihrer pharmazeutisch annehmbaren Salze umfaßt, Gegenstand der Erfindung. Dabei können die erfindungsgemäßen Verbindungen in dem erfindungsgemäßen Arzneimittel als isomerenreine, insbesondere enantiomerenreine bzw. diastereomerenreine, Verbindungen, aber auch als racemisches oder nicht-racemisches Gemisch vorliegen. Bevorzugt ist dabei, daß das Arzneimittel ein pharmazeutisch annehmbares Salz der erfindungsgemäßen Verbindungen enthält, insbesondere ein Hydrochlorid oder ein Natriumsalz.

Ein weiterer Gegenstand der Erfindung ist die Verwendung mindestens einer erfindungsgemäßen Verbindung der allgemeinen Formel (I), einschließlich ihrer Diastereomeren oder Enantiomeren, auch als Racemate oder Enantiomerengemisch, in Form ihrer freien Base oder Säure oder eines mit einer physiologisch verträglichen Säure bzw. Base gebildeten Salzes, insbesondere des Hydrochloridsalzes und des Natriumsalzes, zur Herstellung eines Arzneimittels zur Behandlung von Schmerz. Die erfindungsgemäßen Verbindungen haben sich als analgetisch wirksam erwiesen und weisen eine hohe Affinität zur Bindungsstelle 2 des Natriumkanals (BTX-Bindungsstelle) auf.

Aufgrund der ausgeprägten Bindung der erfindungsgemäßen Verbindungen der allgemeinen Formel (I) an die BTX-Bindungsstelle hat sich auch herausgestellt, daß diese Verbindungen auch für die Verwendung hinsichtlich weiterer Indikationen geeignet sind. Daher ist ein weiterer Gegenstand der vorliegenden Erfindung die Verwendung von Verbindungen der allgemeinen Formel (I) oder von ihren pharmazeutisch annehmbaren Salzen zur Herstellung eines Lokalanästhetikums, eines Antiarrhythmikums, eines Antiemetikums, eines Nootropikums und/oder eines Arzneimittels zur Behandlung und/oder Prophylaxe von cardiovaskulären Erkrankungen, von Harninkontinenz, Diarrhöe, Pruritus und/oder Entzündungen und/oder eines Arzneimittels zur Behandlung von Depressionen und/oder Alkohol- und/oder Drogen- und/oder Medikamentenmißbrauch und/oder eines Arzneimittels zur Vigilanzsteigerung.

Darüber hinaus sind auch pharmazeutische Zusammensetzungen Gegenstand der vorliegenden Erfindung, die mindestens eine Verbindung der wie oben definierten allgemeinen Formel (I) oder eines ihrer pharmazeutisch annehmbaren Salze und einen oder mehrere pharmazeutische Hilfsstoffe enthalten.

Die erfindungsgemäßen Arzneimittel und pharmazeutischen Zusammensetzungen können als flüssige, halbfeste oder feste Arzneiformen und in Form von z.B. Injektionslösungen, Tropfen, Säften, Sirupen, Sprays, Suspensionen, Granulaten, Tabletten, Pellets, transdermalen therapeutischen Systemen, Kapseln, Pflastern, Zäpfchen, Salben, Cremes, Lotionen, Gelen, Emulsionen oder Aerosolen vorliegen und verabreicht werden und enthalten neben mindestens einer erfindungsgemäßen Verbindung der allgemeinen Formel (I) je nach galenischer Form pharmazeutische Hilfsstoffe, wie z.B. Trägermaterialien, Füllstoffe, Lösungsmittel, Verdünnungsmittel, oberflächenaktive Stoffe, Farbstoffe, Konservierungsstoffe, Sprengmittel, Gleitmittel, Schmiermittel, Aromen und/oder Bindemittel. Diese Hilfsstoffe können beispielsweise sein: Wasser, Ethanol, 2-Propanol, Glycerin, Ethylenglycol, Propylenglycol, Polyethylenglycol, Polypropylenglycol, Glucose, Fructose, Lactose, Saccharose, Dextrose, Melasse, Stärke, modifizierte Stärke, Gelatine, Sorbitol, Inositol, Mannitol, mikrokristalline Cellulose, Methylcellulose, Carboxymethylcellulose, Celluloseacetat, Schellack, Cetylalkohol, Polyvinylpyrrolidon, Paraffine, Wachse, natürliche und synthetische Gummis, Akaziengummi, Alginate, Dextran, gesättigte und ungesättigte Fettsäuren, Stearinsäure, Magnesiumstearat, Zinkstearat, Glycerylstearat, Natriumlaurylsulfat, genießbare Öle, Sesamöl, Kokusnußöl, Erdnußöl, Sojabohnenöl, Lecithin, Natriumlactat, Polyoxyethylen- und -propylenfettsäureester, Sorbitanfettsäureester, Sorbinsäure, Benzoesäure, Citronensäure, Ascorbinsäure, Tanninsäure, Natriumchlorid, Kaliumchlorid, Magnesiumchlorid, Calciumchlorid, Magnesiumoxid, Zinkoxid, Siliciumdioxid, Titanoxid, Titandioxid, Magnesiumsulfat, Zinksulfat, Calciumsulfat, Pottasche, Calciumphosphat, Dicalciumphosphat, Kaliumbromid, Kaliumiodid, Talkum, Kaolin, Pectin, Crospovidon, Agar und Bentonit.

Die Auswahl der Hilfsstoffe sowie die einzusetzenden Mengen derselben hängt davon ab, ob das Arzneimittel oral, subkutan, parenteral, intravenös, vaginal, pulmonal, intraperitoneal, transdermal, intramuskulär, nasal, buccal, rectal oder örtlich, zum Beispiel auf der Haut, den Schleimhäuten bzw. an den Augen, appliziert werden soll. Für die orale Applikation eignen sich u.a. Zubereitungen in Form von Tabletten, Dragees, Kapseln, Granulaten, Tropfen, Säften und Sirupen, für die parenterale, topische und inhalative Applikation Lösungen, Suspensionen, leicht rekonstituierbare Pulver zur Inhalation sowie Sprays. Erfindungsgemäße Verbindungen der allgemeinen Formel (I) in einem Depot in gelöster Form oder in einem Pflaster, gegebenenfalls unter Zusatz von die Hautpenetration fördernden Mitteln, sind geeignete perkutane Applikationszubereitungen. Rektal, transmucosal, parenteral, oral oder perkutan anwendbare Zubereitungsformen können die erfindungsgemäßen Verbindungen der allgemeinen Formel (I) verzögert freisetzen.

Die Herstellung der erfindungsgemäßen Arzneimittel und pharmazeutischen Zusammensetzungen erfolgt mit Hilfe von im Stand der Technik der pharmazeutischen Formulierung wohlbekannten Mitteln, Vorrichtungen, Methoden und Verfahren, wie sie beispielsweise in "Remington's Pharmaceutical Sciences", Hrsg. A.R. Gennaro, 17. Ed., Mack Publishing Company, Easton, Pa. (1985), insbesondere in Teil 8, Kapitel 76 bis 93, beschrieben sind.

So kann z.B. für eine feste Formulierung, wie eine Tablette, der Wirkstoff des Arzneimittels, d.h. eine Verbindung der allgemeinen Formel (I) oder eines ihrer pharmazeutisch annehmbaren Salze, mit einem pharmazeutischen Träger, z.B. herkömmlichen Tabletteninhaltsstoffen, wie Maisstärke, Lactose, Saccharose, Sorbitol, Talkum, Magnesiumstearat, Dicalciumphosphat oder pharmazeutisch akzeptable Gummis, und pharmazeutischen Verdünnungsmitteln, wie z.B. Wasser, granuliert werden, um eine feste Zusammensetzung zu bilden, die eine erfindungsgemäße Verbindung oder ein pharmazeutisch annehmbares Salz davon in homogener Verteilung enthält. Unter einer homogenen Verteilung wird hier verstanden, daß der Wirkstoff gleichmäßig über die gesamte Zusammensetzung verteilt ist, so daß diese ohne weiteres in gleich wirksame Einheitsdosis-Formen, wie Tabletten, Pillen oder Kapseln, unterteilt werden kann. Die feste Zusammensetzung wird anschließend in Einheitsdosis-Formen unterteilt. Die Tabletten oder Pillen des erfindungsgemäßen Arzneimittels bzw. der erfindungsgemäßen Zusammensetzungen können auch überzogen oder auf andere Weise kompoundiert werden, um eine Dosisform mit verzögerter Freisetzung bereitzustellen. Geeignete Beschichtungsmittel sind u.a. polymere Säuren und Mischungen von polymeren Säuren mit Materialien wie z.B. Schellack, Cetylalkohol und/oder Celluloseacetat.

Die an den Patienten zu verabreichende Wirkstoffmenge variiert und ist abhängig vom Gewicht, dem Alter und der Krankheitsgeschichte des Patienten, sowie von der Applikationsart, der Indikation und dem Schweregrad der Erkrankung. Üblicherweise werden 0,1 bis 5000 mg/kg, insbesondere 1 bis 500 mg/kg, vorzugsweise 2 bis 250 mg/kg Körpergewicht wenigstens einer erfindungsgemäßen Verbindung der allgemeinen Formel (I) appliziert.

Die nachfolgenden Beispiele dienen zur näheren Erläuterung der vorliegenden Erfindung.

### Beispiele

Die eingesetzten Chemikalien und Lösungsmittel wurden kommerziell von einem der folgenden Anbieter erworben: Acros, Geel; Avocado, Port of Heysham; Aldrich, Deisenhofen; Fluka, Seelze; Lancaster, Mülheim; Maybridge, Tintagel; Merck, Darmstadt; Sigma, Deisenhofen; TCl, Japan; oder nach allgemeinen im Stand der Technik bekannten Verfahren hergestellt.

Die dünschichtchromatographischen Untersuchungen wurden mit HPTLC-Fertigplatten, Kieselgel 60 F 254, der Firma E. Merck, Darmstadt, durchgeführt.

Jede Probe wurde mit ESI-MS und/oder NMR analysiert. Massenspektrometrische Untersuchungen (ESI-MS) wurden mit einem Massenspektrometer der Fa. Finnegan, LCQ Classic durchgeführt. ¹H-NMR-Untersuchungen der erfindungsgemäßen Verbindungen wurden mit einem 300 MHz DPX Advance NMR-Gerät der Fa. Bruker durchgeführt.

### Allgemeine Arbeitsvorschrift 1 (AAV 1)

Ein Rundbodenröhrchen aus Glas (Durchmesser 16 mm, Länge 125 mm) mit Gewinde wurde manuell mit einem Rührer versehen und auf einer Capper-Station mit einem Schraubdeckel mit Septum verschlossen. Das Röhrchen wurde von einem Roboter 1 in einen auf 20°C temperierten Rührblock gestellt. Ein Roboter 2 pipettierte nacheinander die folgenden Reagenzien hinzu:
1. 1 ml einer Lösung, die Trifluoressigsäure und die Aminokomponente jeweils 0,1 M enthielt, in Acetonitril
2. 1 ml einer 0,11 M Aldehyd-Lösung in Acetonitril
3. 1 ml einer 0,3 M Furan/Thiophen-Lösung in Acetonitril.

Das Reaktionsgemisch wurde bei 40°C in einem der Rührblöcke 600 min lang gerührt. Danach wurde die Reaktionslösung an einer Filtrations-Station abfiltriert. Das Röhrchen wurde dabei zweimal mit 1,5 ml einer 7,5% NaHCO₃-Lösung gespült.

Das Rack mit den Proben wurde manuell auf die Aufarbeitungsanlage gestellt. Das Reaktionsgemisch wurde auf einem Vortexer mit 2 ml Ethylacetat versetzt und geschüttelt. Zur Ausbildung der Phasengrenze wurde in der Zentrifuge kurz zentrifugiert. Die Phasengrenze wurde optisch detektiert und die organische Phase abpipettiert. Im nächsten Schritt wurde die wäßrige Phase erneut mit 2 ml Ethylacetat versetzt, geschüttelt, zentrifugiert und die organische Phase abpipettiert. Die vereinigten organischen Phasen wurden über 2,4 g MgSO₄ (granuliert) getrocknet. Das Lösungsmittel wurde in einer Vakuumzentrifuge entfernt. Jede Probe wurde mit ESI-MS und/oder NMR analysiert.

Durch die automatisierte Synthese ist eine Gleichbehandlung aller Proben sowie eine ausgesprochen konstante Reaktionsführung gewährleistet.

Einige Beispielsverbindungen sind in Tabelle 1 wiedergegeben:

**Tabelle 1**

| **Beispiel** | **Name** | **Berechnete Masse** | **Gefundene Masse** |
|---|---|---|---|
| A | 5-[1-(2-Chlor-phenylamino)-2-oxo-2-phenylethyl]-2-methyl-furan-3-carbonsäureethytester | 383,83 | 384,0/386,1 |
| B | 5-[1-(4-Chlor-2-methyl-phenylamino)-2-oxo-2-phenyl-ethyl]-2-methyl-furan-3-carbonsäuremethylester | 397,85 | 398,1/400,2 |
| C | 5-[1-(4-Chlor-2-fluor-phenylamino)-2-oxo-2-phenyl-ethyl]-2-methyl-furan-3-carbonsäuremethylester | 401,82 | 400,4/402,5 |
| D | 5-[1-(4-Chlor-2-methyl-phenylamino)-2-oxo-2-phenyl-ethyl]-2-methyl-furan-3-carbonsäureethylester | 411,88 | 410,3/412,1/ 414,1 |

### Pharmakologische Untersuchungen

### Bindungsuntersuchungen am Natriumkanal - Bindungsstelle 2 (BTX-Bindung):

Die Bindungsstelle 2 des Natriumkanals ist die sogenannte Batrachotoxin-(BTX-)Bindungsstelle. Als Ligand wurde [³H]-Batrachotoxinin A20 α-Benzoat (10 nM im Ansatz) eingesetzt. Diese Ionenkanal-Partikel (Synaptosomen) wurden aus dem Ratten-Cerebrocortex nach Gray und Whittaker (E.G. Gray und V.P. Whittaker, *J. Anat.* (1962) 79-88) angereichert. Als unspezifische Bindung ist die Radioaktivität definiert, die in Gegenwart von Veratridin (0,3 mM im Ansatz) gemessen wird. Die Inkubation erfolgte bei 25°C für 120 min. Die Assaybedingungen entsprachen denen der Veröffentlichung von Pauwels, Leysen und Laduron (P.J. Pauwels, J.E. Leysen und P.M. Laduron, *Eur. J. Pharmacol*. (1986) 291-298).

Der K_{D}-Wert für diese Bindungsstelle liegt bei 24,63 ± 1,56 nM. (N = 2, d.h. Mittelwerte ± SEM aus 2 unabhängigen Versuchsreihen, die in Zweifach-Parallelversuchen durchgeführt worden sind).

Die experimentellen Werte der BTX-Bindung für ausgewählte Beispielsverbindungen sind in Tabelle 2 wiedergegeben:

**Tabelle 2**

| **Beispiel** | **BTX-Bindung (10µM)** |
|---|---|
| C | 57% |
| D | 37% |
| B | 55% |
| A | 40% |

### Pharmazeutische Formulierung eines erfindungsgemäßen Arzneimittels

1 g des Hydrochlorids von 5-[1-(4-Chlor-2-fluor-phenylamino)-2-oxo-2-phenyl-ethyl]-2-methyl-furan-3-carbonsäuremethylester wurde in 1 I Wasser für Injektionszwecke bei Raumtemperatur gelöst und anschließend durch Zugabe von Natriumchlorid auf isotone Bedingungen eingestellt.

## Patentansprüche

1. Verbindung der allgemeinen Formel (I), oder ein pharmazeutisch annehmbares Salz davon, 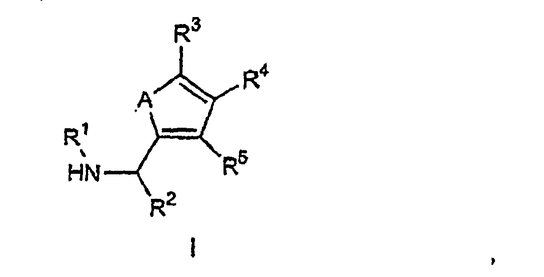 worin
A O oder S bedeutet;
R¹ für 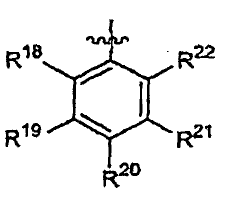 oder für 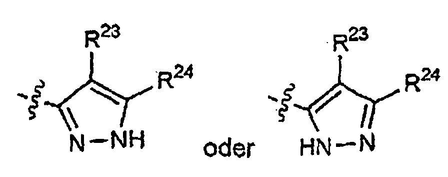 steht;
R² -(C=O)-Phenyl oder -*cyclo*-C₃H₄R¹⁷ bedeutet;
R³, R⁴ und R⁵ unabhängig voneinander H, Methyl, -CH₂-OH, -CH₂-S-CH₃ oder -CH₂-S-CH₂-furan-2-yl, - C(=O)OMethyl, -C(=O)OEthyl, -CH₂-C(=O)OEthyl bedeuten;
R¹⁷ -C(=O)OH oder -C(=O)O-C₁₋₆-Alkyl bedeutet; und
R¹⁸, R¹⁹, R²⁰, R²¹, R²², R²³ und R²⁴ unabhängig voneinander H, OH, SH, -O-C₁₋₆-Alkyl, -OAryl, -S-C₁₋₆-Alkyl, -SAryl, F, Cl, Br, I, -CN, C₁₋₆-Alkyl, CF₃, CO(=O)H, CO(=O)-C₁₋₆-Alkyl oder -N=N-Aryl bedeuten,
in Form ihres Racemats, in Form der reinen Enantiomeren oder Diastereomeren oder in Form von Mischungen der Enantiomeren oder Diastereomeren in einem beliebigen Mischungsverhältnis.

2. Verbindung nach Anspruch 1, **dadurch gekennzeichnet, dass**
R² -(C=O)-Phenyl oder -*cyclo*-C₃H₄-C(=O)OEthyl bedeutet;
R³ H, Methyl, -CH₂-S-CH₃, -CH₂-S-CH₂-furan-2-yl oder -CH₂-C(=O)OEthyl bedeutet;
R⁴ H, Methyl, -CH₂-OH, -C(=O)OMethyl oder -C(=O)OEthyl bedeutet;
R⁵ H bedeutet;
R¹⁸, R¹⁹, R²⁰, R²¹ und R²² unabhängig voneinander H, -OPhenyl, F, Cl, Br, -CN, Methyl und CF₃ bedeuten, wobei mindestens 3 der Reste R¹⁸, R¹⁹, R²⁰, R²¹ und R²² H bedeuten; und
R²³ und R²⁴ unabhängig voneinander H, OH, -S-Methyl, -CN, CO(=O)-Ethyl oder -N=N-Phenyl bedeuten.

3. Verbindung nach Anspruch 1, oder ein pharmazeutisch annehmbares Salz davon, **dadurch gekennzeichnet, daß** sie ausgewählt ist aus:
5-[1-(2-Chlor-phenylamino)-2-oxo-2-phenyl-ethyl]-2-methyl-furan-3-carbonsäureethylester;
5-[1-(4-Chlor-2-methyl-phenylamino)-2-oxo-2-phenyl-ethyl]-2-methylfuran-3-carbonsäuremethylester;
5-[1-(4-Chlor-2-fluor-phenylamino)-2-oxo-2-phenyl-ethyl]-2-methylfuran-3-carbonsäuremethylester; und
5-[1-(4-Chlor-2-methyl-phenylamino)-2-oxo-2-phenyl-ethyl]-2-methylfuran-3-carbonsäureethylester.

4. Verfahren zur Herstellung einer Verbindung der allgemeinen Formel (I), oder eines pharmazeutisch annehmbaren Salzes davon, 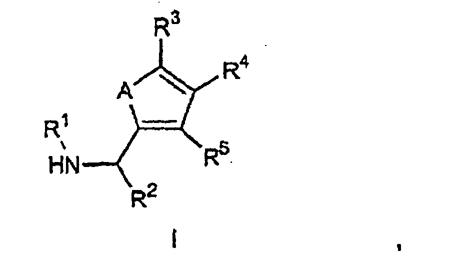 worin
A O oder S bedeutet;
R¹ für 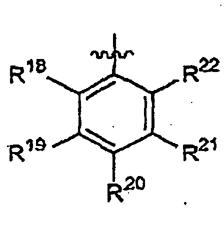 oder für 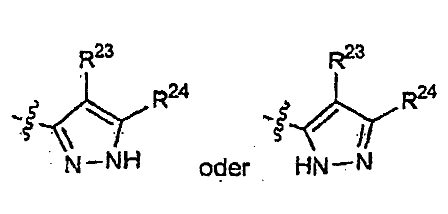 steht;
R² -(C=O)-Phenyl oder -*cyclo*-C₃H₄R¹⁷ bedeutet;
R³, R⁴ und R⁵ unabhängig voneinander H, Methyl, -CH₂-OH, -CH₂-S-CH₃ oder -CH₂-S-CH₂-furan-2-yl, - C(=O)OMethyl, -C(=O)OEthyl, -CH₂-C(=O)OEthyl bedeuten;
R¹⁷ -C(=O)OH oder -C(=O)O-C₁₋₆-Alkyl bedeutet; und
R¹⁸, R¹⁹, R²⁰, R²¹, R²², R²³ und R²⁴ unabhängig voneinander H, OH, SH, -O-C₁₋₆-Alkyl, -OAryl, -S-C₁₋₆-Alkyl, -SAryl, F, Cl, Br, I, -CN, C₁₋₆-Alkyl, CF₃, CO(=O)H, CO(=O)-C₁₋₆-Alkyl oder -N=N-Aryl bedeuten,
in Form ihres Racemats, in Form der reinen Enantiomeren oder Diastereomeren oder in Form von Mischungen der Enantiomeren oder Diastereomeren in einem beliebigen Mischungsverhältnis **dadurch gekennzeichnet, dass**
ein Amin der allgemeinen Formel (II) 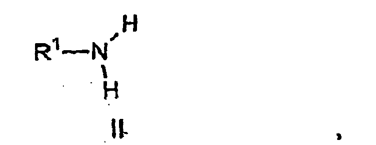 worin R¹ wie oben in diesem Anspruch definiert ist,
mit einem Aldehyd der allgemeinen Formel (III) 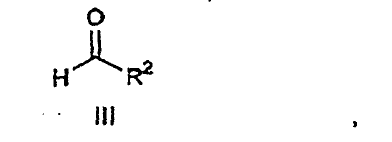 worin R² wie oben in diesem Anspruch definiert ist,
und einem Heterocyclus der allgemeinen Formel (IV) 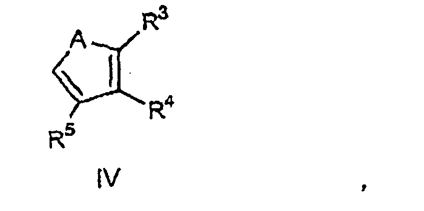 worin A, R³, R⁴ und R⁵ wie oben in diesem Anspruch definiert sind,
in Gegenwart einer Säure umgesetzt werden.

5. Verfahren nach Anspruch 4, **dadurch gekennzeichnet, dass** die Säure Trifluoressigsäure ist.

6. Verfahren nach einem der Ansprüche 4 oder 5, **dadurch gekennzeichnet, dass** die Umsetzung in einem organischen Lösungsmittel und bei einer Temperatur von 0° bis 100° C ausgeführt wird.

7. Arzneimittel enthaltend eine Verbindung der allgemeinen Formel (I) oder eines ihrer pharmazeutisch annehmbaren Salze 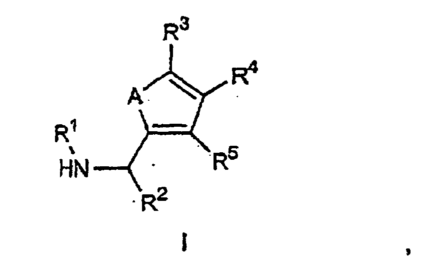 worin
A O oder S bedeutet;
R¹ für 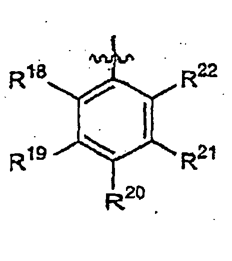 oder für 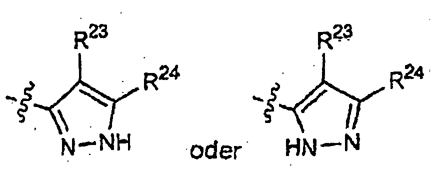 steht;
R² -(C=O)-Phenyl oder -*cyclo*-C₃H₄R¹⁷ bedeutet;
R³, R⁴ und R⁵ unabhängig voneinander H, Methyl, -CH₂-OH, -CH₂-S-CH₃ oder -CH₂-S-CH₂-furan-2-yl, -C(=O)OMethyl, -C(=O)OEthyl, -CH₂-C(=O)OEthyl bedeuten;
R¹⁷ -C(=O)OH oder -C(=O)O-C₁₋₆-Alkyl bedeutet; und
R¹⁸, R¹⁹, R²⁰, R²¹, R²², R²³ und R²⁴ unabhängig voneinander H, OH, SH, -O-C₁₋₆-Alkyl, -OAryl, -S-C₁₋₆-Alkyl, -SAryl, F, Cl, Br, I, -CN, C₁₋₆-Alkyl, CF₃, CO(=O)H, CO(=O)-C₁₋₆-Alkyl oder -N=N-Aryl bedeuten,
in Form ihres Racemats, in Form der reinen Enantiomeren oder Diastereomeren oder in Form von Mischungen der Enantiomeren oder Diastereomeren in einem beliebigen Mischungsverhältnis.

8. Verwendung einer Verbindung der allgemeinen Formel (I) oder eines Ihrer pharmazeutisch annehmbaren Salze 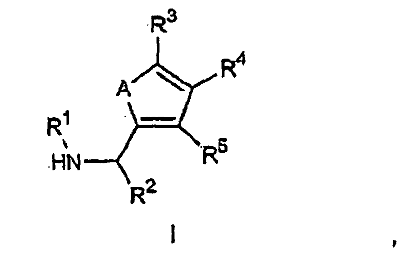 worin
A O oder S bedeutet;
R¹ für 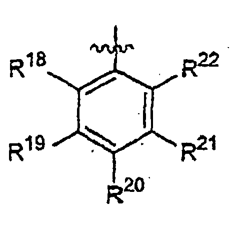 oder für 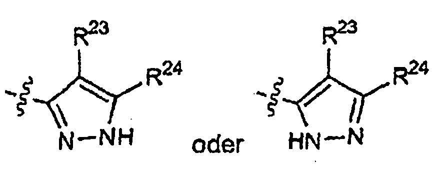 steht;
R² -(C=O)-Phenyl oder -*cyclo*-C₃H₄R¹⁷ bedeutet;
R³, R⁴ und R⁵ unabhängig voneinander H, Methyl, -CH₂-OH, -CH₂-S-CH₃ oder -CH₂-S-CH₂-furan-2-yl, - C(=O)OMethyl, -C(=O)OEthyl, -CH₂-C(=O)OEthyl bedeuten;
R¹⁷ -C(=O)OH oder -C(=O)O-C₁₋₆-Alkyl bedeutet; und
R¹⁸, R¹⁹, R²⁰, R²¹, R²², R²³ und R²⁴ unabhängig voneinander H, OH, SH, -O-C₁₋₆-Alkyl, -OAryl, -S-C₁₋₆-Alkyl, -SAryl, F, Cl, Br, I, -CN, C₁₋₆-Alkyl, CF₃, CO(=O)H, CO(=O)-C₁₋₆-Alkyl oder -N=N-Aryl bedeuten,
in Form ihres Racemats, in Form der reinen Enantiomeren oder Diastereomeren oder in Form von Mischungen der Enantiomeren oder Diastereomeren in einem beliebigen Mischungsverhältnis; zur Herstellung eines Schmerzmittels.

9. Verwendung einer Verbindung der allgemeinen Formel (I) oder eines ihrer pharmazeutisch annehmbaren Salze 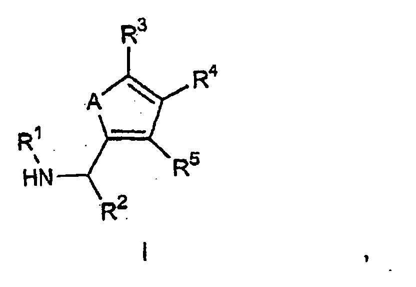 worin
A O oder S bedeutet;
R¹ für 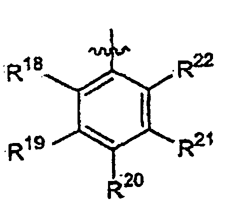 oder für 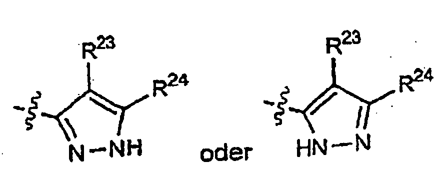 steht;
R² -(C=O)-Phenyl oder *-cyclo*-C₃H₄R¹⁷ bedeutet;
R³, R⁴ und R⁵ unabhängig voneinander H, Methyl, -CH₂-OH, -CH₂-S-CH₃ oder -CH₂-S-CH₂-furan-2-yl, - C(=O)OMethyl, -C(=O)OEthyl, -CH₂-C(=O)OEthyl bedeuten;
R¹⁷ -C(=O)OH oder -C(=O)O-C₁₋₆-Alkyl bedeutet; und
R¹⁸, R¹⁹, R²⁰, R²¹, R²², R²³ und R²⁴ unabhängig voneinander H, OH, SH, -O-C₁₋₆-Alkyl, -OAryl, -S-C₁₋₆-Alkyl, -SAryl, F, Cl, Br, I, -CN, C₁₋₆-Alkyl, CF₃, CO(=O)H, CO(=O)-C₁₋₆-Alkyl oder -N=N-Aryl bedeuten,
in Form ihres Racemats, in Form dar reinen Enantiomeren oder Diastereomeren oder in Form von Mischungen der Enantiomeren oder Diastereomeren in einem beliebigen Mischungsverhältnis zur Herstellung eines Lokalanästhetikums, eines Antiarrhythmikums, eines Antiemetikums, eines Nootropikums und/oder eines Arzneimittels zur Behandlung und/oder Prophylaxe von cardiovaskulären Erkrankungen, von Harninkontinenz, Diarrhöe, Pruritus und/oder Entzündungen und/oder eines Armeimittels zur Behandlung von Depressionen und/oder Alkohol- und/oder Drogen- und/oder Medikamentenmißbrauch und/oder eines Arzneimittels zur Vigilanzsteigerung.

10. Pharmazeutische Zusammensetzung, umfassend
mindestens eine Verbindung der allgemeinen Formel (I) oder eines ihrer pharmazeutisch annehmbaren Salze 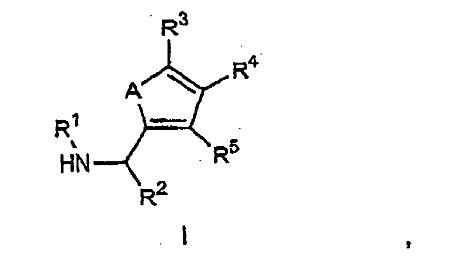 worin
A O oder S bedeutet;
R¹ für 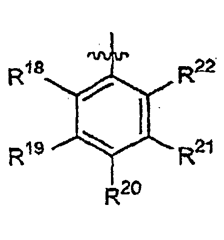 oder für 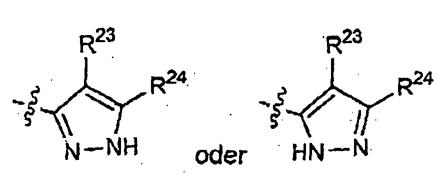 steht;
R² -(C=O)-Phenyl oder -*cyclo*-C₃H₄R¹⁷ bedeutet;
R³, R⁴ und R⁵ unabhängig voneinander H, Methyl, -CH₂-OH, -CH₂-S-CH₃ oder -CH₂-S-CH₂-furan-2-yl, -C(=O)OMethyl, -C(=O)OEthyl, -CH₂-C(=O)OEthyl bedeuten;
R¹⁷ -C(=O)OH oder -C(=O)O-C₁₋₆-Alkyl bedeutet; und
R¹⁸, R¹⁹, R²⁰, R²¹, R²², R²³ und R²⁴ unabhängig voneinander H, OH, SH, -O-C₁₋₆-Alkyl, -OAryl, -S-C₁₋₆-Alkyl, -SAryl, F, Cl, Br, I, -CN, C₁₋₆-Alkyl, CF₃, CO(=O)H, CO(=O)-C₁₋₆-Alkyl oder -N=N-Aryl bedeuten,
in Form ihres Racemats, in Form der reinen Enantiomeren oder Diastereomeren oder in Form von Mischungen der Enantiomeren oder Diastereomeren in einem beliebigen Mischungsverhältnis und mindestens einen pharmazeutischen Hilfsstoff.

## Claims

1. Compound of the general formula (I), or a pharmaceutically acceptable salt thereof, 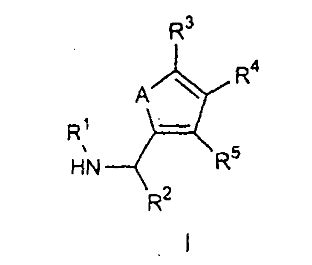 wherein
A represents O or S;
R¹ represents 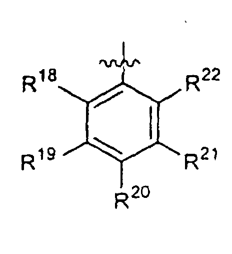 or represents 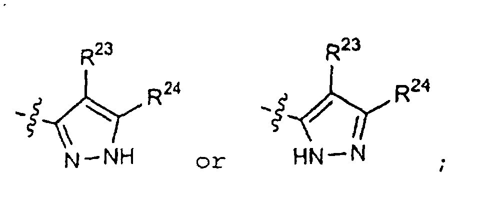
R² represents -(C=O) -phenyl or -*cyclo*-C₃H₄R¹⁷;
R³, R⁴ and R⁵ each independently of the others represents H, methyl, -CH₂-OH, -CH₂-S-CH₃ or -CH₂-S-CH₂-furan-2-yl, -C(=O)Omethyl, -C(=O)Oethyl, -CH₂-C(=O)Oethyl
R¹⁷ represents -C(=O)OH or -C(=O)O-C₁₋₆-alkyl; and
R¹⁸, R¹⁹, R²⁰, R²¹, R²², R²³ and R²⁴ each independently of the others represents H, OH, SH, -O-C₁₋₆-alkyl, -Oaryl, -S-C₁₋₆-alkyl, -Saryl, F, Cl, Br, I, -CN, C₁₋₆-alkyl, CF₃, CO(=O)H, CO(=O)-C₁₋₆-alkyl or -N=N-aryl.
in the form of its racemate, in the form of the pure enantiomers or diastereoisomers, or in the form of mixtures of the enantiomers or diastereoisomers in any desired mixing ratio.

2. Compound according to claim 1, **characterised in that**
R² represents -(C=O)-phenyl or -*cyclo*-C₃H₄-C(=O)Oethyl;
R³ represents H, methyl, -CH₂-S-CH₃, -CH₂-S-CH₂-furan-2-yl or -CH₂-C(=O)Oethyl;
R⁴ represents H, methyl, -CH₂-OH, -C(=O)Omethyl or - C(=O)Oethyl;
R⁵ represents H;
R¹⁸, R¹⁹, R²⁰, R²¹ and R²² each independently of the others represents H, -Ophenyl, F, Cl, Br, -CN, methyl and CF₃, wherein at least three of the radicals R¹⁸, R¹⁹, R²⁰, R²¹ and R²² represent H; and
R²³ and R²⁴ each independently of the other represents H, OH, -S-methyl, -CN, CO(=O)-ethyl or -N=N-phenyl.

3. Compound according to claim 1, or a pharmaceutically acceptable salt thereof, **characterised in that** it is selected from:
5-[1-(2-chloro-phenylamino)-2-oxo-2-phenyl-ethyl]-2-methyl-furan-3-carboxylic acid ethyl ester;
5-[1-(4-chloro-2-methyl-phenylamino)-2-oxo-2-phenylethyl]-2-methyl-furan-3-carboxylic acid methyl ester;
5-[1-(4-chloro-2-fluoro-phenylamino)-2-oxo-2-phenylethyl]-2-methyl-furan-3-carboxylic acid methyl ester; and
5-[1-(4-chloro-2-methyl-phenylamino)-2-oxo-2-phenylethyl]-2-methyl-furan-3-carboxylic acid ethyl ester.

4. Process for the preparation of a compound of the general formula (I), or of a pharmaceutically acceptable salt thereof, 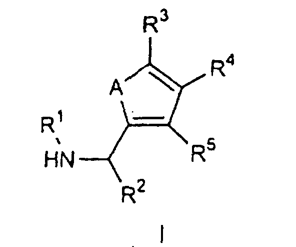 wherein
A represents O or S;
R¹ represents 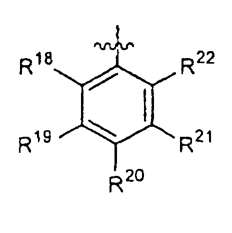 or represents 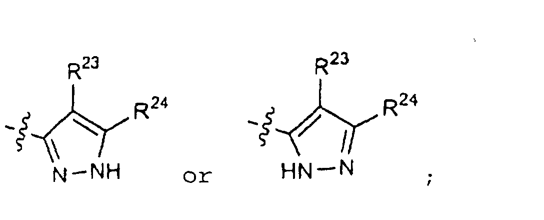
R² represents -(C=O)-phenyl or -*cyclo*-C₃H₄R¹⁷;
R³, R⁴ and R⁵ each independently of the others represents H, methyl, -CH₂-OH, -CH₂-S-CH₃ or -CH₂-S-CH₂-furan-2-yl, -C(=O)Omethyl, -C(=O)Oethyl, - CH₂-C(=O)Oethyl;
R¹⁷ represents -C(=O)OH or -C(=O)O-C₁₋₆-alkyl; and
R¹⁸, R¹⁹, R²⁰, R²¹, R²², R²³ and R²⁴ each independently of the others represents H, OH, SH, -O-C₁₋₆-alkyl, -Oaryl, -S-C₁₋₆-alkyl, -Saryl, F, Cl, Br, I, -CN, C₁₋₆-alkyl, CF₃, CO(=O)H, CO(=O)-C₁₋₆-alkyl or -N=N-aryl.
in the form of its racemate, in the form of the pure enantiomers or diastereoisomers, or in the form of mixtures of the enantiomers or diastereoisomers in any desired mixing ratio, **characterised in that**
an amine of the general formula (II) 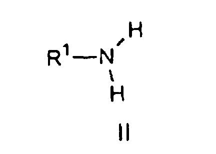 wherein R¹ is as defined above in this claim,
is reacted in the presence of an acid with an aldehyde of the general formula (III) 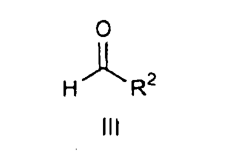 wherein R² is as defined above in this claim,
and with a heterocycle of the general formula (IV) 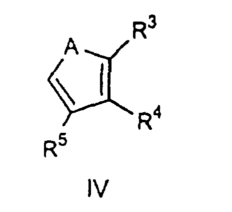 wherein A, R³, R⁴ and R⁵ are as defined above in this claim.

5. Process according to claim 4, **characterised in that** the acid is trifluoroacetic acid.

6. Process according to either claim 4 or claim 5, **characterised in that** the reaction is carried out in an organic solvent and at a temperature of from 0° to 100°C.

7. Medicament containing a compound of the general formula (I) or a pharmaceutically acceptable salt thereof 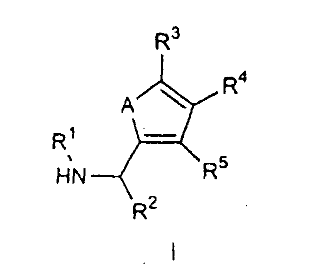 wherein
A represents O or S;
R¹ represents 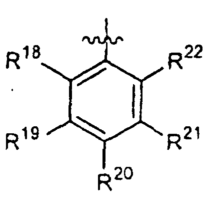 or represents 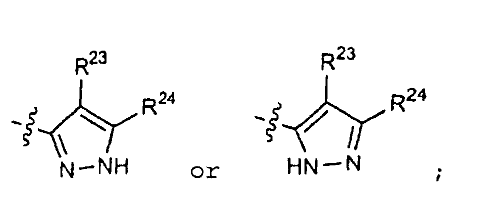
R² represents -(C=O) -phenyl or -*cyclo*-C₃H₄R¹⁷;
R³, R⁴ and R⁵ each independently of the others represents H, methyl, -CH₂-OH, -CH₂-S-CH₃ or -CH₂-S-CH₂-furan-2-yl, -C(=O)Omethyl, -C(=O)Oethyl, - CH₂-C(=O)Oethyl;
R¹⁷ represents -C(=O)OH or -C(=O)O-C₁₋₆-alkyl; and
R¹⁸, R¹⁹, R²⁰, R²¹, R²², R²³ and R²⁴ each independently of the others represents H, OH, SH, -O-C₁₋₆-alkyl, -Oaryl, -S-C₁₋₆-alkyl, -Saryl, F, Cl, Br, I, -CN, C₁₋₆-alkyl, CF₃, CO(=O)H, CO(=O)-C₁₋₆-alkyl or -N=N-aryl.
in the form of its racemate, in the form of the pure enantiomers or diastereoisomers, or in the form of mixtures of the enantiomers or diastereoisomers in any desired mixing ratio.

8. Use of a compound of the general formula (I) or of a pharmaceutically acceptable salt thereof 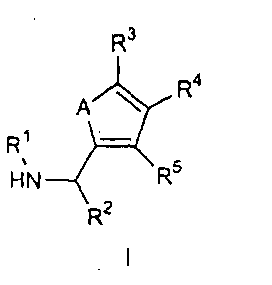 wherein
A represents O or S;
R¹ represents 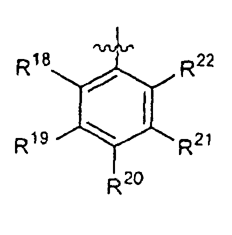 or represents 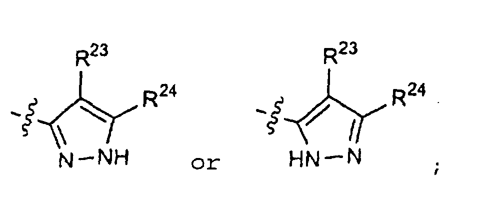
R² represents -(C=O) -phenyl or -*cyclo*-C₃H₄R¹⁷;
R³, R⁴ and R⁵ each independently of the others represents H, methyl, -CH₂-OH, -CH₂-S-CH₃ or -CH₂-S-CH₂-furan-2-yl, -C(=O)Omethyl, -C(=O)Oethyl, - CH₂-C(=O)Oethyl;
R¹⁷ represents -C(=O)OH or -C(=O)O-C₁₋₆-alkyl; and
R¹⁸, R¹⁹, R²⁰, R²¹, R²², R²³ and R²⁴ each independently of the others represents H, OH, SH, -O-C₁₋₆-alkyl, -Oaryl, -S-C₁₋₆-alkyl, -Saryl, F, Cl, Br, I, -CN, C₁₋₆-alkyl, CF₃, CO(=O)H, CO(=O)-C₁₋₆-alkyl or -N=N-aryl.
in the form of its racemate, in the form of the pure enantiomers or diastereoisomers, or in the form of mixtures of the enantiomers or diastereoisomers in any desired mixing ratio; in the preparation of an analgesic.

9. Use of a compound of the general formula (I) or of a pharmaceutically acceptable salt thereof 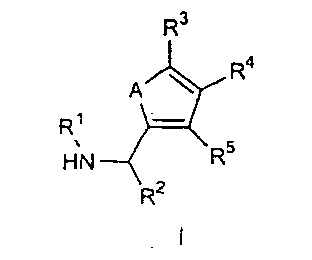 wherein
A represents O or S;
R¹ represents 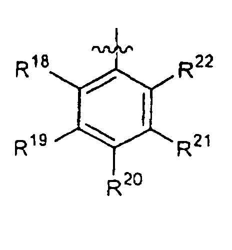 or represents 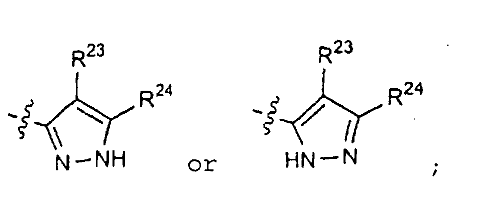
R² represents -(C=O) -phenyl or -*cyclo*-C₃H₄R¹⁷;
R³, R⁴ and R⁵ each independently of the others represents H, methyl, -CH₂-OH, -CH₂-S-CH₃ or -CH₂-S-CH₂-furan-2-yl, -C(=O)Omethyl, -C(=O)Oethyl, -CH₂-C(=O)Oethyl;
R¹⁷ represents -C(=O)OH or -C(=O)O-C₁₋₆-alkyl; and
R¹⁸, R¹⁹, R²⁰, R²¹, R²², R²³ and R²⁴ each independently of the others represents H, OH, SH, -O-C₁₋₆-alkyl, -Oaryl, -S-C₁₋₆-alkyl, -Saryl, F, Cl, Br, I, -CN, C₁₋₆-alkyl, CF₃, CO(=O)H, CO(=O)-C₁₋₆-alkyl or -N=N-aryl,
in the form of its racemate, in the form of the pure enantiomers or diastereoisomers, or in the form of mixtures of the enantiomers or diastereoisomers in any desired mixing ratio, in the preparation of a local anaesthetic, an antiarrhythmic, an antiemetic, a nootropic agent and/or a medicament for the treatment and/or prophylaxis of cardiovascular diseases, urinary incontinence, diarrhoea, pruritus and/or
inflammations, and/or a medicament for the treatment of depression and/or alcohol and/or drug and/or medicament abuse, and/or a medicament for increasing vigilance.

10. Pharmaceutical composition containing at least one compound of the general formula (I) or a pharmaceutically acceptable salt thereof 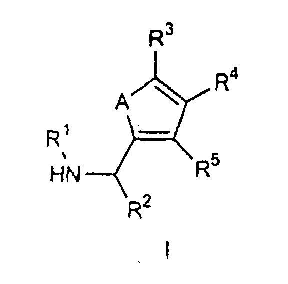 wherein
A represents O or S;
R¹ represents 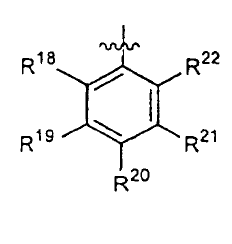 or represents 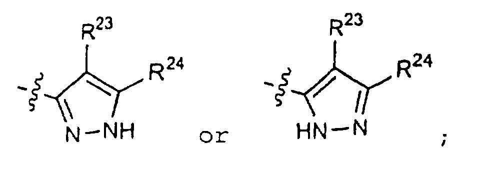
R² represents -(C=O) -phenyl or -*cyclo*-C₃H₄R¹⁷;
R³, R⁴ and R⁵ each independently of the others represents H, methyl, -CH₂-CH, -CH₂-S-CH₃ or -CH₂-S-CH₂-furan-2-yl, -C(=O)Omethyl, -C(=O)Oethyl, -CH₂-C(=O)Oethyl;
R¹⁷ represents -C(=O)OH or -C(=O)O-C₁₋₆-alkyl; and
R¹⁸, R¹⁹, R²⁰, R²¹, R²², R²³ and R²⁴ each independently of the others represents H, OH, SH, -O-C₁₋₆-alkyl, -Oaryl, -S-C₁₋₆-alkyl, -Saryl, F, Cl, Br, I, -CN, C₁₋₆-alkyl, CF₃, CO(=O)H, CO(=O)-C₁₋₆-alkyl or -N=N-aryl.
in the form of its racemate, in the form of the pure enantiomers or diastereoisomers, or in the form of mixtures of the enantiomers or diastereoisomers in any desired mixing ratio, and at least one pharmaceutical excipient.

## Revendications

1. Composé de formule générale (I), ou un sel acceptable du point de vue pharmaceutique de ce composé, 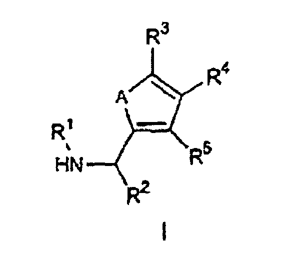 formule dans laquelle
A représente O ou S ;
R¹ représente 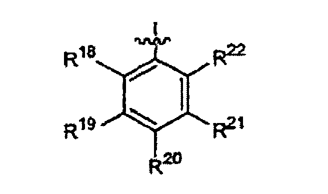 ou 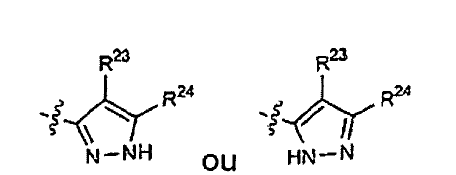
R² représente un reste -(C=O)-phényle ou -*cyclo*-C₃H₄R¹⁷ ;
R³, R⁴ et R⁵ représentent, indépendamment les uns des autres, H, un reste méthyle, -CH₂-OH, -CH₂-S-CH₃ ou -CH₂-S-CH₂-furanne-2-yle, -C(=O)Ométhyle, -C(=O)-Oéthyle, -CH₂-C(=O)Oéthyle ;
R¹⁷ représente un groupe -C(=O)OH ou -C(=O)O-alkyle en C₁ à C₆ ; et
R¹⁸, R¹⁹, R²⁰, R²¹, R²², R²³ et R²⁴ représentent, indépendamment les uns des autres, H, un groupe OH, SH, un reste -O-alkyle en C₁ à C₆, -Oaryle, -S-alkyle en C₁ à C₆, -Saryle, F, Cl, Br, I, un groupe -CN, un reste alkyle en C₁ à C₆, CF₃, CO(=O)H, CO(=O)-alkyle en C₁ à C₆ ou -N=N-aryle,
sous forme de son racémate, sous forme des énantiomères ou des diastéréoisomères purs, ou sous forme de mélanges des énantiomères ou des diastéréoisomères dans un rapport de mélange quelconque.

2. Composé suivant la revendication 1, **caractérisé en ce que**
R² représente un reste -(C=O)-phényle ou -*cyclo*-C₃H₄-C(=O)Oéthyle ;
R³ représente H, un reste méthyle, -CH₂-S-CH₃, -CH₂-S-CH₂-furanne-2-yle ou -CH₂-C(=O)Oéthyle ;
R⁴ représente H, un reste méthyle, -CH₂-OH, -C(=O)-Ométhyle ou -C(=O)Oéthyle ;
R⁵ représente H ;
R¹⁸, R¹⁹, R²⁰, R²¹ et R²² représentent, indépendamment les uns des autres, H, un reste -Ophényle, F, Cl, Br, un groupe -CN, un reste méthyle et un reste CF₃, au moins trois des restes R¹⁸, R¹⁹, R²⁰, R²¹ et R²² représentent H ; et
R²³ et R²⁴ représentent, indépendamment l'un de l'autre, H, un groupe OH, un reste S-méthyle, un groupe -CN, un reste CO(=O)-éthyle ou -N=N-phényle.

3. Composé suivant la revendication 1 ou un sel acceptable du point de vue pharmaceutique de ce composé, **caractérisé en ce qu'**il est choisi entre :
l'ester éthylique d'acide 5-[1-(2-chlorophénylamino)-2-oxo-2-phényl-éthyl]-2-méthylfuranne-3-carboxylique ;
l'ester méthylique d'acide 5-[1-(4-chloro-2-méthylphénylamino)-2-oxo-2-phényléthyl]-2-méthylfuranne-3-carboxylique ;
l'ester méthylique d'acide 5-[1-(4-chloro-2-fluorophénylamino)-2-oxo-2-phényléthyl]-2-méthylfuranne-3-carboxylique ; et
l'ester éthylique d'acide 5-[1-(4-chloro-2-méthylphénylamino)-2-oxo-2-phényléthyl]-2-méthylfuranne-3-carboxylique.

4. Procédé de production d'un composé de formule générale (I) ou d'un sel acceptable du point de vue pharmaceutique de ce composé, 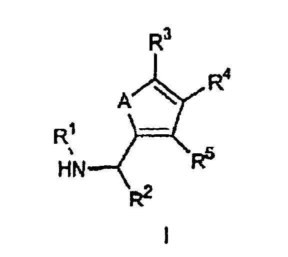 formule dans laquelle
A représente O ou S ;
R¹ représente 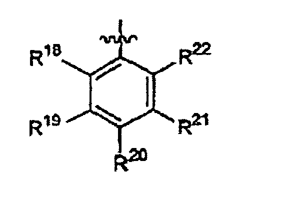 ou 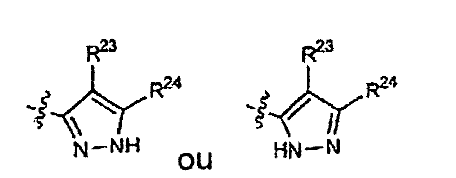
R² représente un reste -(C=O)-phényle ou -*cyclo*-C₃H₄R¹⁷ ;
R³, R⁴ et R⁵ représentent, indépendamment les uns des autres, H, un reste méthyle, -CH₂-OH, -CH₂-S-CH₃ ou -CH₂-S-CH₂-furanne-2-yle, -C(=O)Ométhyle, -C(=O)-Oéthyle, -CH₂-C(=O)Oéthyle ;
R¹⁷ représente un groupe -C(=O)OH ou -C(=O)O-alkyle en C₁ à C₆ ; et
R¹⁸, R¹⁹, R²⁰, R²¹, R²², R²³ et R²⁴ représentent, indépendamment les uns des autres, H, un groupe OH, SH, un reste -O-alkyle en C₁ à C₆, -Oaryle, -S-alkyle en C₁ à C₆, -Saryle, F, Cl, Br, I, un groupe -CN, un reste alkyle en C₁ à C₆, CF₃, CO(=O)H, CO(=O)-(alkyle en C₁ à C₆) ou -N=N-aryle,
sous forme de son racémate, sous forme des énantiomères ou des diastéréoisomères purs, ou sous forme de mélanges des énantiomères ou des diastéréoisomères dans un rapport de mélange quelconque,
**caractérisé en ce qu'**on fait réagir une amine de formule générale (II) 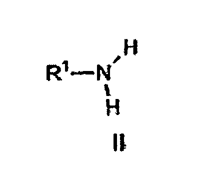 dans laquelle R¹ est tel que défini ci-dessus dans cette revendication,
avec un aldéhyde de formule générale (III) 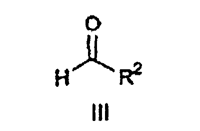 dans laquelle R² est tel que défini ci-dessus dans cette revendication,
et un hétérocycle de formule générale (IV) 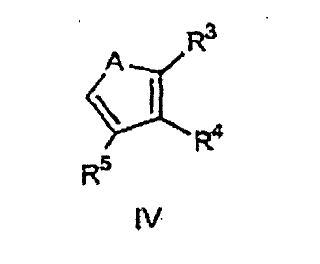 dans laquelle A, R³, R⁴ et R⁵ sont tels que définis ci-dessus dans cette revendication, en présence d'un acide.

5. Procédé suivant la revendication 4, **caractérisé en ce que** l'acide est l'acide trifluoracétique.

6. Procédé suivant l'une des revendications 4 ou 5, **caractérisé en ce que** la réaction est conduite dans un solvant organique et à une température de 0° à 100°C.

7. Médicament contenant un composé de formule générale (I) ou un de ses sels acceptables du point de vue pharmaceutique 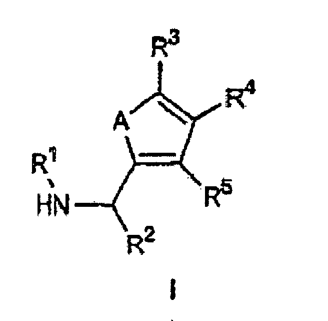 dans laquelle
A représente O ou S ;
R¹ représente 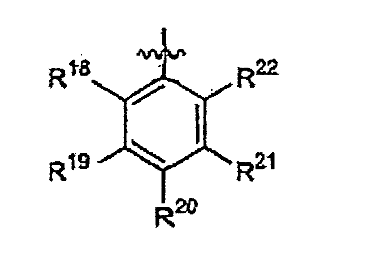 ou 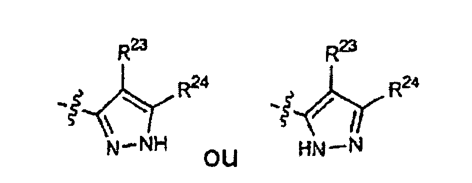
R² représente un reste -(C=O)-phényle ou *-cyclo*-C₃H₄R¹⁷ ;
R³, R⁴ et R⁵ représentent, indépendamment les uns des autres, H, un reste méthyle, -CH₂-OH, -CH₂-S-CH₃ ou -CH₂-S-CH₂-furanne-2-yle, -C(=O)Ométhyle, -C(=O)-Oéthyle, -CH₂-C(=O)Oéthyle ;
R¹⁷ représente un groupe -C(=O)OH ou -C(=O)O-alkyle en C₁ à C₆ ; et
R¹⁸, R¹⁹, R²⁰, R²¹, R²², R²³ et R²⁴ représentent, indépendamment les uns des autres, H, un groupe OH, SH, un reste -O-alkyle en C₁ à C₆, -Oaryle, -S-alkyle en C₁ à C₆, -Saryle, F, Cl, Br, I, un groupe -CN, un reste alkyle en C₁ à C₆, CF₃, CO(=O)H, CO(=O)-alkyle en C₁ à C₆ ou -N=N-aryle,
sous forme de son racémate, sous forme des énantiomères ou des diastéréoisomères purs, ou sous forme de mélanges des énantiomères ou des diastéréoisomères dans un rapport de mélange quelconque.

8. Utilisation d'un composé de formule générale (I) ou de l'un de ses sels acceptables du point de vue pharmaceutique 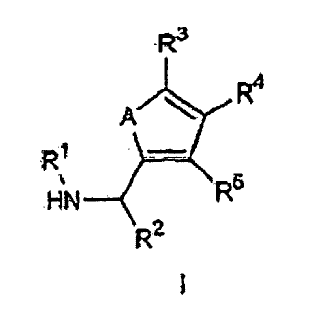 formule dans laquelle
A représente O ou S ;
R¹ représente 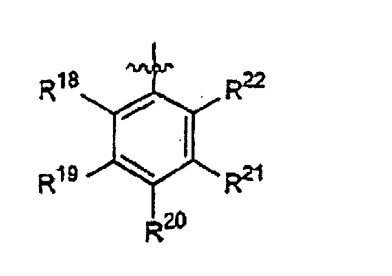 ou 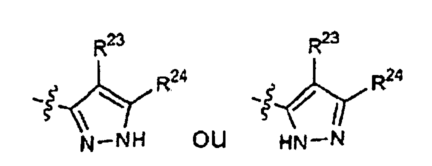
R² représente un reste -(C=O)-phényle ou -*cyclo*-C₃H₄R¹⁷ ;
R³, R⁴ et R⁵ représentent, indépendamment les uns des autres, H, un reste méthyle, -CH₂-OH, -CH₂-S-CH₃ ou -CH₂-S-CH₂-furanne-2-yle, -C(=O)Ométhyle, -C(=O)-Oéthyle, -CH₂-C(=O)Oéthyle ;
R¹⁷ représente un groupe -C(=O)OH ou -C(=O)O-alkyle en C₁ à C₆ ; et
R¹⁸, R¹⁹, R²⁰, R²¹, R²², R²³ et R²⁴ représentent, indépendamment les uns des autres, H, un groupe OH, SH, un reste -O-alkyle en C₁ à C₆, -Oaryle, -S-alkyle en C₁ à C₆, -Saryle, F, Cl, Br, I, un groupe -CN, un reste alkyle en C₁ à C₆, CF₃, CO(=O)H, CO(=O)-(alkyle en C₁ à C₆) ou -N=N-aryle,
sous forme de son racémate, sous forme des énantiomères ou des diastéréoisomères purs, ou sous forme de mélanges des énantiomères ou des diastéréoisomères dans un rapport de mélange quelconque pour la préparation d'un antalgique.

9. Utilisation d'un composé de formule générale (I) ou de l'un de ses sels acceptables du point de vue pharmaceutique 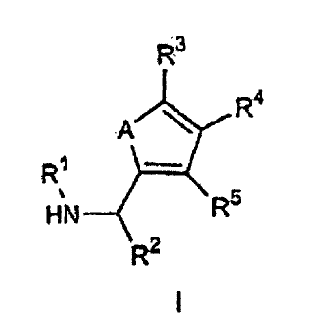 formule dans laquelle
A représente O ou S ;
R¹ représente 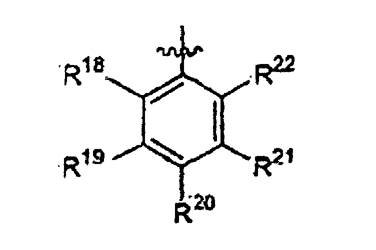 ou 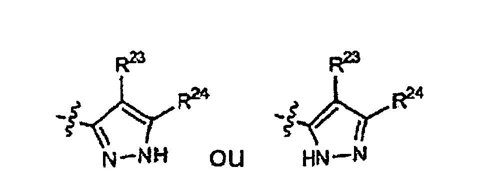
R² représente un reste -(C=O)-phényle ou -*cyclo*-C₃H₄R¹⁷ ;
R³, R⁴ et R⁵ représentent, indépendamment les uns des autres, H, un reste méthyle, -CH₂-OH, -CH₂-S-CH₃ ou -CH₂-S-CH₂-furanne-2-yle, -C(=O)Ométhyle, -C(=O)-Oéthyle, -CH₂-C(=O)Oéthyle ;
R¹⁷ représente un groupe -C(=O)OH ou -C(=O)O-alkyle en C₁ à C₆ ; et
R¹⁸, R¹⁹, R²⁰, R²¹, R²², R²³ et R²⁴ représentent, indépendamment les uns des autres, H, un groupe OH, SH, un reste -O-alkyle en C₁ à C₆, -Oaryle, -S-alkyle en C₁ à C₆, -Saryle, F, Cl, Br, I, un groupe -CN, un reste alkyle en C₁ à C₆, CF₃, CO(=O)H, CO(=O)-alkyle en C₁ à C₆ ou -N=N-aryle,
sous forme de son racémate, sous forme des énantiomères ou des diastéréoisomères purs, ou sous forme de mélanges des énantiomères ou des diastéréoisomères dans un rapport de mélange quelconque pour la préparation d'un anesthésique local, d'un agent anti-arythmique, d'un anti-émétique, d'un nootrope et/ou d'un médicament destiné au traitement et/ou à la prophylaxie de maladies cardio-vasculaires, de l'incontinence urinaire, de la diarrhée, du prurit et/ou d'inflammations, et/ou d'un médicament destiné au traitement de dépressions et/ou d'usage abusif de l'alcool et/ou de drogues et/ou de médicaments, et/ou d'un médicament destiné à accroître la vigilance.

10. Préparation pharmaceutique, comprenant au moins un composé de formule générale (I) ou l'un de ses sels acceptables du point de vue pharmaceutique 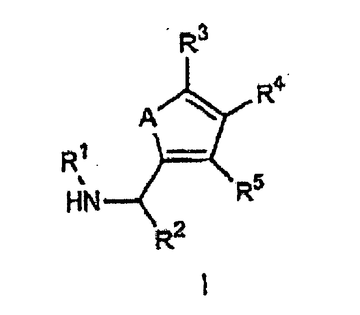 formule dans laquelle
A représente O ou S ;
R¹ représente 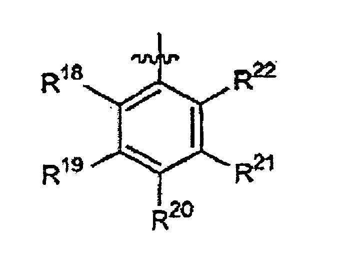 ou 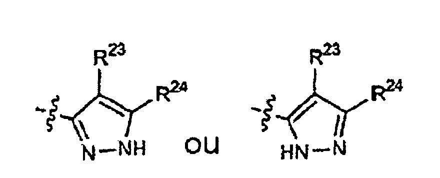
R² représente un reste -(C=O)-phényle ou -*cyclo*-C₃H₄R¹⁷ ;
R³, R⁴ et R⁵ représentent, indépendamment les uns des autres, H, un reste méthyle, -CH₂-OH, -CH₂-S-CH₃ ou -CH₂-S-CH₂-furanne-2-yle, -C(=O)Ométhyle, -C(=O)-Oéthyle, -CH₂-C(=O)Oéthyle ;
R¹⁷ représente un groupe -C(=O)OH ou -C(=O)O-alkyle en C₁ à C₆ ; et
R¹⁸, R¹⁹, R²⁰, R²¹, R²², R²³ et R²⁴ représentent, indépendamment les uns des autres, H, un groupe OH, SH, un reste -O-alkyle en C₁ à C₆, -Oaryle, -S-alkyle en C₁ à C₆, -Saryle, F, Cl, Br, I, un groupe -CN, un reste alkyle en C₁ à C₆, CF₃, CO(=O)H, CO(=O)-alkyle en C₁ à C₆ ou -N=N-aryle,
sous forme de son racémate, sous forme des énantiomères ou des diastéréoisomères purs, ou sous forme de mélanges des énantiomères ou des diastéréoisomères dans un rapport de mélange quelconque, et au moins une substance pharmaceutique auxiliaire.
